Europäisches Patentamt

European Patent Office

Office européen des brevets

(19)

(11) Veröffentlichungsnummer : **0 138 190**
**B1**

(12)  **EUROPÄISCHE PATENTSCHRIFT**

(45) Veröffentlichungstag der Patentschrift :
19.07.89

(51) Int. Cl.⁴ : **G 01 N 33/48**

(21) Anmeldenummer : 84112146.0

(22) Anmeldetag : 10.10.84

(54) Anordnung zur Messung der ex-vivo-Blutungszeit.

(30) Priorität : 15.10.83 DE 3337618

(43) Veröffentlichungstag der Anmeldung :
24.04.85 Patentblatt 85/17

(45) Bekanntmachung des Hinweises auf die Patenterteilung : 19.07.89 Patentblatt 89/29

(84) Benannte Vertragsstaaten :
AT CH FR GB LI

(56) Entgegenhaltungen :
DE--A-- 1 598 732
US--A-- 2 312 488

(73) Patentinhaber : Völkl, Klaus-Peter, Dr.
Robert-Koch-Strasse 28
D-4400 Münster (DE)

(72) Erfinder : Völkl, Klaus-Peter, Dr.
Robert-Koch-Strasse 28
D-4400 Münster (DE)
Erfinder : Schröer, Heinz, Prof. Dr.
Ahornallee 7
D-4400 Münster (DE)

(74) Vertreter : Hofmann, Hans Walter, Dr.
Hauptstrasse 38
D-6549 Tiefenbach (DE)

**Beschreibung**

Die Erfindung betrifft eine Anordnung zur Messung der Aggregation von Blutplaettchen.

Der Verschluss von Verletzungen im Gefaessystem von Organismen, die einen Blutkreislauf haben, erfolgt weitgehend autonom. Dabei aggregieren die in der Blutfluessigkeit vorhandenen Blutplaettchen und bilden mit Fibrin zusammen einen Pfropf, der die Oeffnung verschliesst und der das Geruest fuer das spaeter nachwachsende, den endgueltigen Verschluss bildende Bindegewebe bildet.

Bisher war angenommen worden, dass die Aggregation von Blutplaettchen ausschliesslich durch das Freiwerden von Zwischensubstanzen, vor allem von Mediatoren und Enzymen entsteht, deren Bildung oder Aktivierung durch verletzte Zellen oder durch die veraenderte Haemodynamik provoziert wird.

Aus der DE-A-1598732 ist bekannt, die Koagulation in ihrem zeitlichen Verlauf durch eine Messung des elektrischen Widerstandes zu bestimmen. Dabei ist die Bestimmung der Blutplaettchenaggregation nicht moeglich, weil diese durch die anderen Summanden des Koagulationsvorganges ueberdeckt wird.

Auch die Anordnung nach US-PS 2312488 gestattet nur die integrale Bestimmung der Blutgerinnung. Dabei wird die Fliessfaehigkeit des Blutes gemessen.

Es ist nun gefunden worden, dass Scherkraefte, wie sie beispielsweise in stroemenden Fluessigkeiten in Wandnaehe entstehen, bei hohen Werten bereits fuer sich eine Aggregation von Blutplaettchen ausloesen.

Diese Erkenntnis gestattet die Messung der Aggregationsfaehigkeit von Blutplaettchen bei einer Blutprobe dadurch, dass eine Blutprobe ueber einer bestimmten in einer Membran angeordneten Messoeffnung, deren Durchmesser das das 0.01-bis 10-fache der Membrandicke betraegt, unter Druck gesetzt und die Zeit gemessen wird, in der eine bestimmte Verringerung der Messöffnung eingetreten ist.

Die Erfindung betrifft eine Anordnung gemäß Anspruch 1.

Die Anordnung zeichnet sich dadurch aus, dass die Plättchenaggregation ohne Beeinflussung durch externe, physiologisch oder pharmakologisch wirksame Substanzen abläuft. Dadurch lassen sich Vergleichswerte für verschiedene Individuen oder verschiedene Zustände des gleichen Individuums gewinnen. Dabei gehen Hämatokrit und Blutviskosität in die Messung ein, so dass diese, die Mikrozirkulation wesentlich bestimmenden Parameter individuell berücksichtigt sind. Andererseits wird das gemessene Ergebnis im wesentlichen durch die Funktion der Thrombozyten bestimmt, so dass nunmehr ein Thrombozytentest zur Verfügung steht, der eine einfache Quantifizierung zulässt.

Reproduzierbare Ergebnisse Lassen sich besonders dann erreichen, wenn der Versuchsraum zu beiden Seiten der Messöffnung feucht gehalten ist, da dann keine Artefakte durch den Trocknungsprozess entstehen.

Die Blutungszeit kann durch bestimmte Krankheiten, beispielsweise Diabetes, oder durch therapeutisch verabreichte Gerinnungsmittel oder Antigerinnungsmittel verändert sein. Mit der Anordnung nach der Erfindung ist eine einfache Überprüfung des jeweiligen Status ermöglicht.

in der Zeichnung sind verschiedene Ausführungen der Erfindung dargestellt. Es zeigen

Fig. 1 : eine vollständige Anordnung mit optischer Bestimmung des Normpunktes,

Fig. 2 : ein Messergebnis

Fig. 3 : eine vergrösserte Darstellung der Messöffnung,

Fig. 4 : eine erste Anordnung mit elektrischer Bestimmung des Normpunktes

Fig. 5 : eine zweite Anordnung mit elektrischer Bestimmung des Normpunktes.

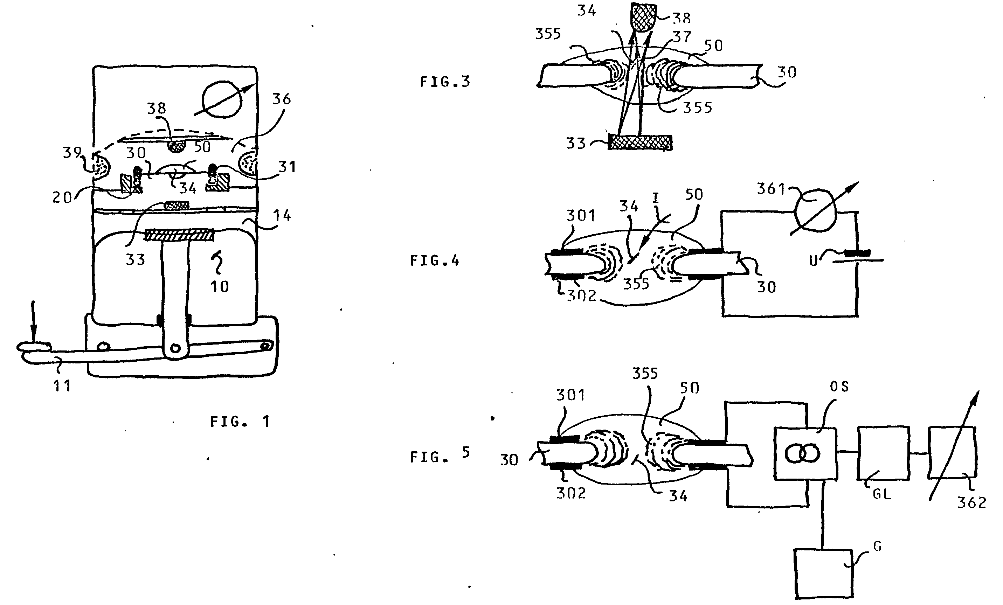

Eine mechanisch spannbare Membranpumpe 10 weist in Fig. 1 an ihrem oberen Ende eine mit einer Dichtung 20 versehene Öffnung auf, in die ein eine Membran 30 tragender Ring 31 einsetzbar ist. Die Membran von beispielsweise 200 μm Dicke weist eine Messöffnung 34 von etwa 0,05 mm Durchmesser auf. Ein Blutstropfen 50 wird auf die Membran 30 über der Messöffnung 34 aufgebracht, der Ring 31 auf die Dichtung 20 gelegt und die Pumpe 10 durch Druck auf den Hebel 11 und anschliessende Arretierung evakuiert. Die Druckdifferenz über der Messöffnung beträgt danach etwa 100 mm Hg.

Der Blutstropfen 50, der in einer beispielsweise durch befeuchtete Watte 39 wasserdampfgesättigten Atmosphäre des Meßkammer 36 angeordnet ist, beginnt, durch die Messöffnung 34 in den unter Niederdruck stehenden Raum 14 der Pumpe 10 auszutreten. Die nunmehr wirksamen Scherkräfte veranlassen dabei eine Aggregation der Blutplättchen. Dadurch wird die Messöffnung 34 verkleinert. Diese Verringerung der Messöffnung wird bestimmt.

Stehen Pressluftinstallationen oder Pressluftflaschen zur Verfügung, kann die Membranpumpe entfallen.

Vorteilhaft ist auch die Thermostatisierung der Messkammer, insbesondere auf eine Temperatur von etwa 310 K.

Die Grösse des Durchmessers der Messöffnung und die Membrandicke stehen im Verhältnis 0.01 bis 10. Die Variation kann dabei zweckmässig auf geringste durchtretende Blutmengen optimiert sein. So etwa, wenn der Durchmesser etwa 25 μm und die Membrandicke etwa 100 μm beträgt.

Ein mit dieser Messmethode gewonnenes Ergebnis zeigt Fig. 2

Hierin wurde die Blutungszeit von normalen Versuchspersonen (a), nach Verabreichung von 6.25 μg Ketanserin pro ml Blut (b) und von Diabetikern (c), nach Verabreichung von 6.25 μg Ketanserin (d) bestimmt.

In Fig. 3 ist die durchbohrte Membran 30 mit der Messöffnung 34 vergrössert dargestellt.

Die Leuchtdiode 33 sendet ihr Licht 37 durch die mit dem Blutstropfen 50 überdeckte Messöffnung 34 der Membran 30, das durch einen Photoempfänger 38 gemessen und durch eine Anzeige 361 (Fig. 4) angezeigt wird.

Die Druckdifferenz über der Länge der die Messöffnung darstellenden Bohrung führt zu der gewünschten Scherkraft, die sich wie folgt berechnet:

Es ist mit

r = Radius der Messöffnung
i Fliessmenge
l = Länge der Messöffnung
dp = Druckdifferenz
Q = Querschnitt der Messöffnung
$\bar{v}$ = Mittlere Fliessgeschwindigkeit
$\mathcal{M}$ = Viskosität
$\gamma$ = Scherrate
$\tau$ = Scherkraft
V = volumen

(1)

$$\tau = \mathcal{M} \cdot \gamma$$

mit (2)

$$\mathcal{M} = \frac{\pi \cdot dp \cdot r^4}{8\,l} \cdot \frac{t}{V}$$

Mit der Scherrate

(3)

$$\gamma = \frac{4\,\bar{v}}{r}$$

ergibt sich

(4)

$$\tau = \frac{\pi \cdot dp.r^3}{2\,l} \cdot \frac{t}{V}\,\bar{v}$$

Wegen

(5)

$$\frac{V}{t} = \bar{v} \cdot Q$$

folgt für die Scherkraft

(6)

$$\tau = \frac{dp \cdot r}{2 \cdot l}$$

Bei den gewählten Messgrössen:

r($\mu$m) = 25
dp (N/m$^2$) = 9600
l (cm) = 0.02
q(cm$^2$) = 1.96*10 exp-5
v(cm/s) 7.4-4.9*10 exp-3

ergibt sich eine Scherkraft von etwa

600 N/m$^2$

Der Lichtdurchsatz durch die Messöffnung 34 wird dadurch verringert, dass die aggregierten Blutplättchen 355 die Messöffnung 34 verkleinern.

Der Endpunkt der Messung ist dann erreicht, wenn kein Blut mehr durch die Messöffnung 34 tritt. Die dazu erforderliche Zeit ist die gesuchte Messgrösse.

Es kann aber auch eine bestimmte Zeit vorgegeben und die danach eingetretene Lumenänderung der Messöffnung festgestellt werden.

Ein solche Methode hat den Vorteil, dass die Bestimmung des Endpunktes genauer ist, weil der Vorgang nicht bis zum Stillstand des Blutdurchflusses geführt ist.

in Fig. 4 ist die Lumenänderung durch eine Leitfähigkeitsmessung bestimmt. Dazu ist die Membran 30 beidseitig metallisiert (301, 302) und eine Gleichspannung U zwischen die Elektroden gelegt. Der elektrische Strom I fliesst ausschliesslich durch die Messöffnung 34. Die Lumenverringerung durch Aggregation 355 kann durch das die Widerstandsänderung anzeigende Messinstrument 361 gut bestimmt werden.

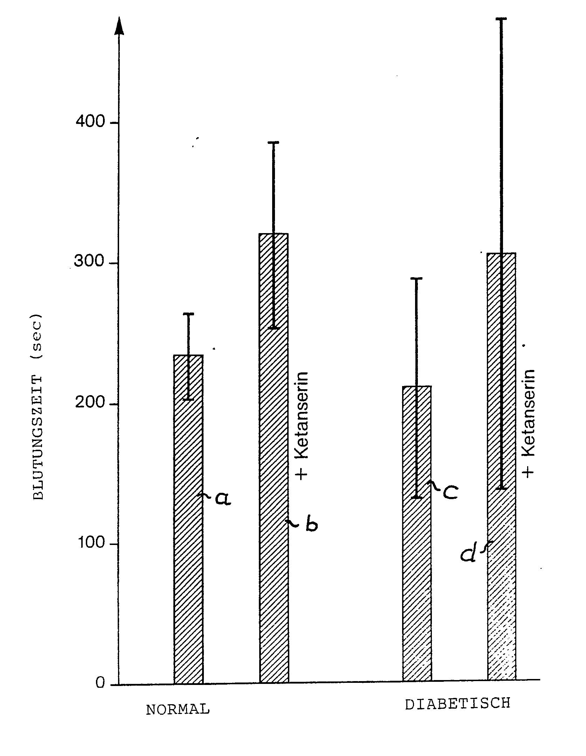

Gemäss Fig. 5 kann die Veränderung der Messöffnung auch dadurch bestimmt werden, dass die Elektroden 301, 302 als kapazitive Schwingkreiselemente verwendet werden. Durch die Aggregation 355 der Blutplättchen ändert sich die Resonanzkurve des Oszillators OS. Diese Änderung wird nach Überlagerung mit dem Signal eines Festoszillators G und Gleichrichtung GL mit dem Messinstrument 362 gemessen.

## Patentansprüche

1. Anordnung zur Messung der Aggregation von Blutplaettchen, dadurch gekennzeichnet, dass sie eine Membran (30) mit einer Messoeffnung (34), deren Durchmesser das 0.01-bis 10-fache der Membrandicke betraegt, aufweist, wobei in der Anordnung eine Blutprobe (50) über der Meßöffnung unter Druck gesetzt und die Zeit gemessen wird, in der eine bestimmte Verringerung der Messoeffnung (34) durch Aggregation (355) von Blutplaettchen eingetreten ist.

2. Anordnung nach Anspruch 1, dadurch gekennzeichnet, dass der Durchmesser der Messoeffnung (34) etwa 0,050 mm betraegt.

3. Anordnung nach Anspruch 1 oder 2, dadurch gekennzeichnet, dass die Anordnung in einer Messkammer (36, 14) angeordnet ist, die eine Wasserdampfsaettigung von etwa 100 % aufweist.

4. Anordnung nach einem der Ansprueche 1 bis 3, dadurch gekennzeichnet, dass die Membran (30) als Nichtleiter ausgebildet und beidseitig metallisiert ist und dass die Aenderung der Leitfaehigkeit ueber der Messoeffnung (34) bestimmt wird.

5. Anordnung nach einem der Ansprueche 1 bis 3, dadurch gekennzeichnet, dass die Membran (30) als Nichtleiter ausgebildet und beidseitig metallisiert ist und dass die Kapazitaetsaenderung ueber der Messoeffnung (34) mittels eines HF-Generators (OS) bestimmt wird.

6. Anordnung nach einem der Ansprueche 1 bis 3, dadurch gekennzeichnet, dass eine Lichtquelle (33) auf der einen Seite, ein Lichtempfaenger (38) auf der anderen Seite der Messoeffnung (34) angeordnet ist und dass der die Messoeffnung (34) durchstrahlende Anteil des Lichts (37) gemessen und angezeigt wird.

7. Anordnung nach einem der Ansprueche 1 bis 6, dadurch gekennzeichnet, dass die zur Messung erforderliche Druckdifferenz durch eine Handpumpe (10) erzeugt ist.

8. Anordnung nach einem der Ansprueche 1 bis 7, dadurch gekennzeichnet, dass die Dicke der Membran (30) etwa 200 $\mu$m betraegt.

9. Anordnung nach einem der Ansprueche 3 bis 8, dadurch gekennzeichnet, dass die Messkammer (36, 14) thermostatisiert ist.

10. Anordnung nach Anspruch 9, dadurch gekennzeichnet, dass die Temperatur etwa 310 K betraegt.

## Claims

1. An arrangement for measuring the aggregation of thrombocytes, characterised in that it has a membrane (30) with a measuring aperture (34) whose diameter is 0.01 to 10 times the thickness of the membrane, wherein a blood sample (50) is placed over the measuring aperture in the arrangement under pressure, and the time is measured in which a definite reduction in the measuring aperture (34) occurs by aggregation (355) of thrombocytes.

2. An arrangement according to claim 1, characterised in that the diameter of the measuring aperture (34) is about 0.050 mm.

3. An arrangement according to claim 1 or 2, characterised in that the apparatus is arranged in a measuring chamber (36, 14) which has a water vapour saturation of about 1000/0.

4. An arrangement according to any of claims 1 to 3, characterised in that the membrane (30) is constructed as a non-conductor and is metallised on both sides and that the change in conductivity across the measuring aperture (34) is defined.

5. An arrangement according to any of claims 1 to 3, characterised in that the membrane (30) is constructed as a non-conductor and is metallised on both sides and that the change in capacitance across the measuring aperture (34) is defined by means of an HF-generator (OS).

6. An arrangement according to any of claims 1 to 3, characterised in that a light source (33) is arranged on one side of the measuring aperture (34) and a light receiver (38) is arranged on the other side of the measuring aperture (34) and that the proportion of light (37) passing through the measuring aperture (34) is measured and recorded.

7. An arrangement according to any of claims 1 to 6, characterised in that the difference in pressure necessary for measuring is produced by means of a hand pump (10).

8. An arrangement according to any of claims 1 to 7, characterised in that the thickness of the membrane (30) is about 200 μm.

9. An arrangement according to any of claims 3 to 8, characterised in that the measuring chamber (36, 14) has a thermostat.

10. An arrangement according to claim 9, characterised in that the temperature is about 310 K.

## Revendications

1. Dispositif pour la mesure de l'aggrégation de plaquettes sanguines, caractérisé par le fait qu'il comprend une membrane (30) avec une ouverture de mesure (34) dont le diamètre est compris entre 0,01 et 10 fois l'épaisseur de la membrane, un échantillon de sang (50) étant, dans le dispositif mis sous pression au-dessus de l'ouverture de mesure et le temps étant mesuré pendant lequel une réduction déterminée de l'ouverture de mesure (34) s'est produite par agrégation (355) de plaquettes sanguines.

2. Dispositif suivant la revendication 1, caractérisé par le fait que le diamètre de l'ouverture de mesure (34) est d'environ 0,050 mm.

3. Dispositif suivant la revendication 1 ou 2, caractérisé par le fait que le dispositif est disposé dans une chambre de mesure (34, 14) qui présente une saturation de vapeur d'eau d'environ 100 %.

4. Dispositif suivant l'une des revendications 1 à 3, caractérisé par le fait que la membrane (30) est réalisée sous forme de non-conducteur et est métallisée sur les deux faces et que la variation de la conductibilité par l'ouverture de mesure (34) est déterminée.

5. Dispositif suivant l'une des revendications 1 à 3, caractérisé par le fait que la membrane (30) est réalisée sous forme de non-conducteur et est métallisée sur les deux faces et que la variation de capacité par l'ouverture de mesure (34) est déterminée au moyen d'un générateur HF (OS).

6. Dispositif suivant l'une des revendications 1 à 3, caractérisé par le fait qu'une source de lumière (33) est disposée sur un côté et un récepteur de lumière (38) est disposé sur l'autre côté de l'ouverture de mesure (34) et que la partie de la lumière (34) traversant l'ouverture de mesure (34) est mesurée et indiquée.

7. Dispositif suivant l'une des revendications 1 à 6, caractérisé par le fait que la différence de pression nécessaire pour la mesure est produite au moyen d'une pompe à main (10).

8. Dispositif suivant l'une des revendications 1 à 7, caractérisé par le fait que l'épaisseur de la membrane (30) ,est d'environ 200 μm.

9. Dispositif suivant l'une des revendications 3 à 8, caractérisé par le fait que la chambre de mesure (36 ; 14) est thermostatée.

10. Dispositif suivant la revendication 3, caractérisé par le fait que la température s'élève à environ 310 K.

FIG. 3

FIG. 4

FIG. 5

FIG. 1